# EUROPEAN PATENT APPLICATION

(11) **EP 3 566 654 A1**
(43) Date of publication of application: **13.11.2019**
(21) Application number: 17894927.7
(22) Date of filing: 03.11.2017
(51) Int. Cl.: A61B 8/00

(54) **ULTRASONIC PROBE AND CONTROL METHOD THEREFOR**

(30) Priority: 06.02.2017 KR 20170016238
(71) Applicant: Samsung Medison Co., Ltd., Hongcheon-gun, Gangwon-do 25108 (KR)
(72) Inventor: JANG, Jing Sung, Incheon 21457 (KR); KIM, Nam Yun, Seoul 04974 (KR)
(74) Representative: Jacobs, Bart
(86) International application number: PCT/KR2017/012407
(87) International publication number: WO 2018/143542

(57) **Abstract**

According to the disclosed aspect, the ultrasonic probe provides the information of the target object, and by adjusting the scalpel provided on the ultrasonic probe based on the information, an ultrasonic probe and controlling method thereof provides to perform a precise incision for each layer of the target object and to detect the main part under the incision for preparing the dangerous situation.

According to one embodiment of the present disclosure includes an incision unit configured to incise a target object, an adjustment unit configured to adjust the incision unit, a transducer configured to irradiate the ultrasonic signal to the target object and receive the reflected ultrasonic signal, and a controller configured to determine the inside of the target object based on the delivered signal by the transducer and control the adjustment unit corresponding to the inside of the target object.

## Description

### [Technical Field]

The disclosed invention is an ultrasonic probe including a scalpel, and relates to a control method of controlling scalpels based on ultrasonic waves received by the probe.

### [Background Art]

Surgery, despite its long history, was not recognized as a very effective treatment until the 20th century. This was due to bleeding and postoperative infection.

In particular, bleeding was not only life-threatening to a patient's life, but also obstructed ambulatory surgery by interfering with a physician's vision during surgery.

In addition, the common opinion of surgeons performing surgery using a scalpel is that when illumination placed in an operating room is obscured by an operator depending on the situation, it is highly likely to make a mistake because the surgeon cannot judge the incision site.

As a result, the surgeons who performed the surgery had to incise a target object by sensation, and there were problems of incising important blood vessels during elaborate depth adjustment operations resulting in accidents.

### [Disclosure]

### [Technical Problem]

According to the disclosed aspect, the ultrasonic probe provides information of the target object, and by adjusting the scalpel provided on the ultrasonic probe based on the information, the ultrasonic probe and controlling method thereof provides to perform a precise incision for each layer of the target object and to detect a main part under the incision in preparation for a dangerous situation.

### [Technical Solution]

In accordance with one embodiment of the present disclosure, an ultrasonic probe may include an incision unit configured to incise a target object, an adjustment unit configured to adjust the incision unit, a transducer configured to irradiate an ultrasonic signal to the target object and receive the reflected ultrasonic signal; and a controller configured to determine the inside of the target object based on the delivered signal by the transducer and control the adjustment unit corresponding to the inside of the target object.

The controller may set a region of interest in which the inside of the target object is incised.

The controller may control the adjustment unit based on a depth of the inside of the target object.

The controller may control the adjustment unit to adjust an angle of the incision unit based on an incision direction of the inside of the target object.

The adjustment unit may further include a piston configured to be connected to the incision unit; and the controller may control hydraulic or air pressure inside the adjustment unit to move the piston.

The adjustment unit may include a rack configured to be connected to the incision unit; a pinion configured to be located on the rack, and rotate; and a motor configured to rotate the pinion; and the controller may control the adjustment unit to adjust the depth of the incision unit by driving the motor.

The incision unit may be disposed at one end of a housing of the ultrasonic probe.

The incision unit may be located at the center of a cover housing of the ultrasonic probe.

The ultrasonic probe may further include a display unit configured to display an adjusting result of the incision unit.

The ultrasonic probe may further include an input unit to receive a user's input command, and the controller may control the adjustment unit based on the delivered signal from the input unit.

In accordance with another aspect of the present disclosure, a method for controlling an ultrasonic probe, the method may include irradiating an ultrasonic signal to a target object and receiving the reflected ultrasonic signal; determining the inside of the target object based on the received ultrasonic signal; and adjusting an adjustment unit corresponding to the inside of the target object.

The determining may set a region of interest in which the inside of the target object is incised.

The adjusting may adjust a depth of the incision unit.

The adjusting may adjust an angle of the adjustment unit based on an incision direction of the target object.

The adjusting may adjust at least one of hydraulic pressure, air pressure and a motor for moving a piston connected to the incision unit.

### [Advantageous Effects]

The ultrasonic probe according to one aspect provides information of the target object and by adjusting the scalpel provided on the ultrasonic probe according to the information, a precise incision can be made for each layer of the object, and a major part can be detected under the incision to prepare for the risk of incision.

### [Description of Drawings]

FIGS. 1 and 2 are external views of an ultrasonic diagnostic apparatus and an ultrasonic probe in accordance with one embodiment.
FIG. 3 is a block diagram of an ultrasonic probe in accordance with an embodiment of the disclosure.
FIGS. 4A and 4B are views illustrating an operation of an ultrasonic probe in accordance with one embodiment.
FIGS. 5A and 5B are views illustrating a method of adjusting an incision unit in accordance with one embodiment.
FIGS. 6A and 6B are views illustrating a method of adjusting an incision unit in accordance with another embodiment.
FIG. 7 is a flowchart of a method of adjusting a depth of an incision unit by an ultrasonic probe in accordance with one embodiment.
FIG. 8 is a flowchart of a method of adjusting an angle of an incision unit by an ultrasonic probe in accordance with one embodiment.
FIG. 9 is a view illustrating an example in which the disclosed ultrasonic probe according to another embodiment is applied to a surgical robot system.
FIG. 10 is a view illustrating an operation of a robot arm.

### [Best Mode]

### [Mode for Invention]

Reference will now be made in detail to the embodiments of the present disclosure, examples of which are illustrated in the accompanying drawings, wherein like reference numerals refer to like elements throughout. This specification does not describe all elements of the embodiments of the present disclosure and detailed descriptions on what are well known in the art or redundant descriptions on substantially the same configurations may be omitted. The terms 'unit, module, member, and block' used herein may be implemented using a software or hardware component. According to an embodiment, a plurality of 'units, modules, members, or blocks' may also be implemented using an element and one 'unit, module, member, or block' may include a plurality of elements.

Throughout the specification, when an element is referred to as being "connected to" another element, it may be directly or indirectly connected to the other element and the "indirectly connected to" includes being connected to the other element via a wireless communication network.

Also, it is to be understood that the terms "include" and "have" are intended to indicate the existence of elements disclosed in the specification, and are not intended to preclude the possibility that one or more other elements may exist or may be added.

Throughout the specification, when a member is located "on" another member, this includes not only when a member is in contact with another member but also when another member is present between the two members.

In this specification, terms "first," "second," etc. are used to distinguish one component from other components and, therefore, the components are not limited by the terms.

An expression used in the singular encompasses the expression of the plural, unless it has a clearly different meaning in the context.

The reference numerals used in operations are used for descriptive convenience and are not intended to describe the order of operations and the operations may be performed in a different order unless otherwise stated.

Hereinafter, embodiments of the present disclosure will be described in detail with reference to the accompanying drawings.

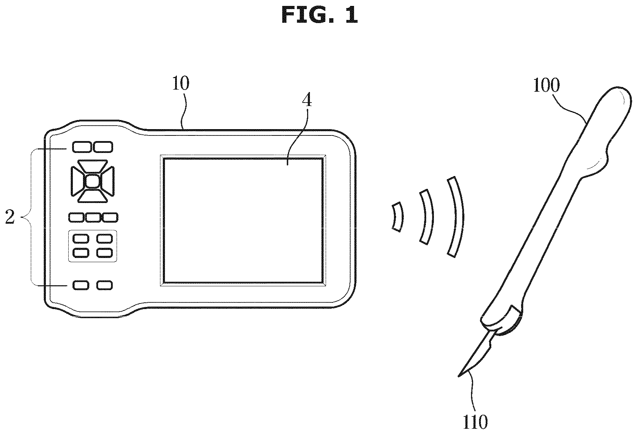

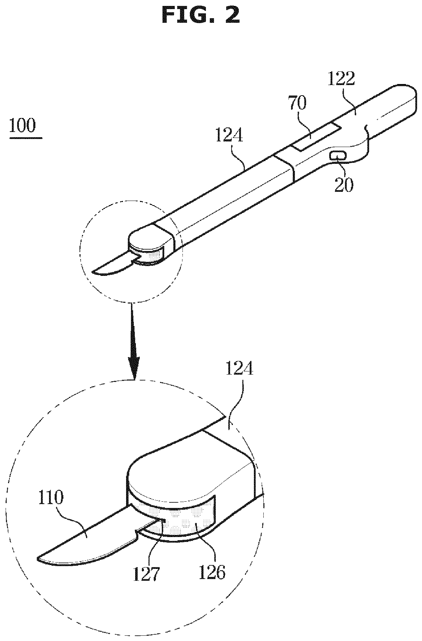

FIGS. 1 and 2 are external views of an ultrasonic diagnosis device and an ultrasonic probe in accordance with one embodiment. To avoid redundant explanations, the following will be described together.

Referring to FIG. 1, an ultrasonic probe 100 may include an ultrasonic diagnostic apparatus 10 that includes an incision unit 110 and generates an image of a target object based on an ultrasonic signal received by the ultrasonic probe 100.

The ultrasonic diagnostic apparatus 10 receives the ultrasonic signal reflected from the target object and received by the ultrasonic probe 100. The ultrasonic diagnostic apparatus 10 can generate an image inside the object based on the received ultrasonic signal.

According to the example shown in FIG. 1, the ultrasonic probe 100 and the ultrasonic diagnostic apparatus 10 exchange signals through wireless communication. However, this is merely an example, and the ultrasonic diagnostic apparatus 10 and the ultrasonic probe 100 may be connected through a wired communication.

On the other hand, the external appearance of the ultrasonic diagnostic apparatus 10 includes a main body input unit 2 for receiving a user's input command and a main body display unit 4 for outputting an image of the object.

The main body input unit 2 receives the user's input command. For example, the user can input an ultrasonic diagnosis start command, a diagnosis selection mode such as an A-mode (Amplitude mode), a B-mode (Brightness mode), a color mode (Color mode), a D-mode (Doppler mode), and an M-mode (Motion mode), and Region of Interest (Region of interest : ROI) setting information including a size and position of the ROI.

The main body input unit 2 includes hardware devices such as various buttons or switches, a pedal, a keyboard, a mouse, a track-ball, various levers, a handle, and a stick.

It is also possible to receive the user's input command through a graphical user interface (GUI) such as a touch pad provided with the main body display unit 4, and it is possible to have a mutual layer structure with the main body display unit 4.

The main body display unit 4 outputs an image of the object based on a signal transmitted by the ultrasonic probe 100. In addition, the main body display unit 4 may output various interfaces for guiding the user so as to change the generated images, or may output various interfaces for controlling the ultrasonic probe 100 together.

The main body display unit 4 included in the main body of the ultrasonic diagnostic apparatus 10 may be a cathode ray tube (CRT), a digital light processing (DLP) panel, a plasma display panel, a liquid crystal display (LCD) panel, an electroluminescence (EL) panel, an electrophoretic display (EPD) panel, an electrochromic display (ECD) panel, a light emitting diode (LED) panel or an organic light emitting diode (OLED) panel, but the present disclosure is not limited thereto.

The ultrasonic diagnostic apparatus 10 includes a processor for controlling the overall operation of the ultrasonic diagnostic apparatus 10. The processor generates an ultrasonic image and controls the main body display unit 4 to output an image.

The processor may also instruct the ultrasonic probe 100 to determine and control a depth or angle of the incision unit 110 included in the ultrasonic probe 100 corresponding to the inside of the object.

Hereinafter, an example in which the ultrasonic probe 100 adjusts the incision unit 110 will be described, but the ultrasonic diagnostic apparatus 10 shown in FIG. 1 may also control the incision unit. In this case, the processor included in the ultrasonic diagnostic apparatus 10 may transmit a control command to adjust the incision unit.

The ultrasonic probe 100 according to the disclosed embodiment includes the incision unit 110.

The incision unit 110 may be used to incise a target, and may include various incisors such as a scalpel used by a physician during surgery or a knife used for meat processing.

Specifically, the incision unit 110 may include a knife for incising a target by the user's force, and a type of incision by generating a high temperature to the target object by using electric and high-frequency waves. One example of various aspects of the incision unit 110 will be described below with reference to other figures.

The incision unit 110 may protrude outward from the inside of the housing, and the depth of the incision unit 110 protruded by a power unit provided inside the ultrasonic probe 100 may be adjusted. In addition, the incision unit 110 can be moved by the power unit to adjust a protruded angle.

The incision unit 110 is adjusted based on the inside of the object determined by the ultrasonic signal received by the ultrasonic probe 100. The method of adjusting the incision unit 110 will be described in detail with reference to the drawings.

FIG. 2 is a view for explaining an external view of the ultrasonic probe including the incision unit 110.

Referring to FIG. 2, a housing 120 of the ultrasonic probe 100 according to an embodiment includes a lower housing 122, an upper housing 124, a cover housing 126, and the incision unit 110 protruded by a central groove 128 of the cover housing 126.

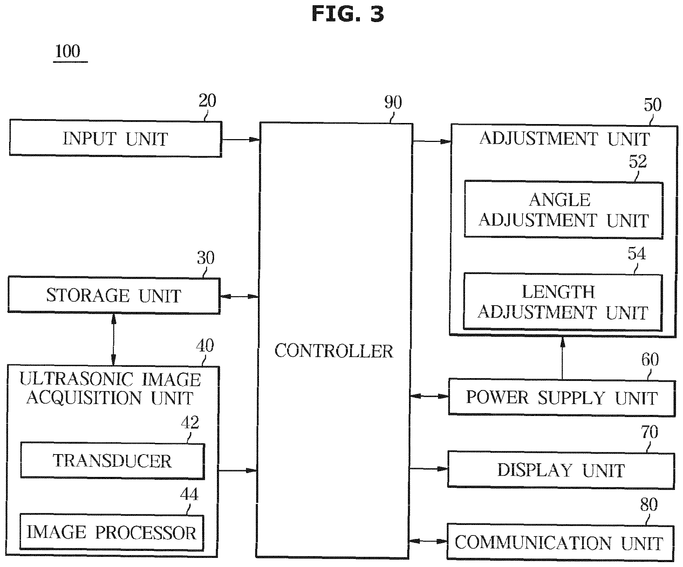

A hardware structure for the basic operation of the ultrasonic probe 100 and a power supply unit 60 (see FIG. 3) for driving the ultrasonic probe 100 may be included in the lower housing 122. For example, the power supply unit 60 receives power from a portable power supply unit including a battery or the like. As another example, when the ultrasonic probe 100 is connected with the ultrasonic diagnostic apparatus 10 in a wired manner, the power supply unit 60 may refer to a module that receives electric power from the ultrasonic diagnostic apparatus 10.

The lower housing 122 may include an input unit 20 for receiving the user's input command and a display unit 70 for displaying the degree to which the incision unit 110 is adjusted. A detailed description thereof will be explained later with reference to FIG. 3.

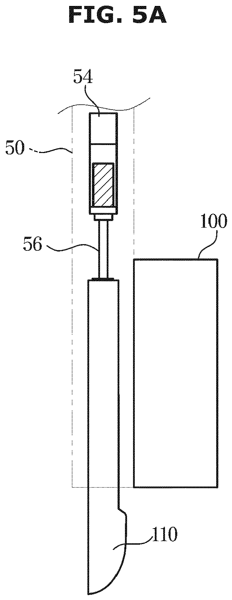

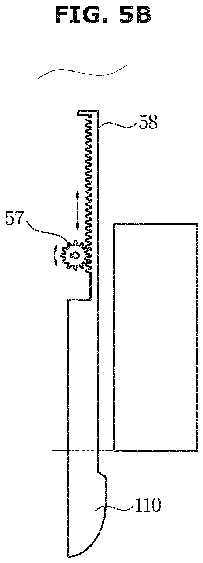

Inside the upper housing 124, an adjustment unit 50 for operating the incision unit 110 may be provided. The adjustment unit 50 may include various actuators for moving the incision unit 110 or adjusting the angle. A detailed description related to the adjustment unit 50 will be described later in detail with reference to FIGS. 5A and 5B.

The cover housing 126 may be provided with an ultrasonic image acquisition unit 40 (see FIG. 3) necessary for acquiring ultrasonic images.

The ultrasonic image acquisition unit 40 includes a transducer 42 for irradiating an object with ultrasonic signals and receiving reflected ultrasonic signals, and an image processing unit 44 for generating a signal for generating an image of a target object by changing the ultrasonic signal received by the transducer 42 into an electrical signal.

Although not specifically shown in FIG. 2, the transducer 42 may be formed as a single-layer structure or a multilayered structure. The transducer 42 can be made of a piezoelectric material having a piezoelectric effect that generates a voltage when mechanical pressure is applied and mechanical strain when a voltage is applied. The upper surface of the transducer 42 may be provided with a matching layer for reducing a difference in acoustic impedance between the transducer 42 and the object, and the lower surface of the transducer 42 may be provided with a sound absorbing layer or the like for absorbing ultrasonic waves passing through the transducer 42.

The incision unit 110 may be configured to pass through a groove 127 provided in the cover housing 126. That is, the incision unit 110 may protrude out of the cover housing 126 by the adjustment unit 50 provided in the upper housing 124. The adjustment unit 50 may adjust the angle of the incision unit 110 by rotating a portion of the cover housing 126 where the groove 127 is provided.

On the other hand, FIG. 2 shows an example in which the incision unit 110 protrudes through the groove 127 provided in the ultrasonic probe 100. However, it is not necessary that the incision unit 110 is necessarily protruded from the inside of the cover housing 126, and it may be separately provided on the side of the ultrasonic probe 100 together with the adjustment unit 50. A detailed description related thereto will be described later with reference to FIGS. 5A and 5B.

FIG. 3 is a block diagram of an ultrasonic probe in accordance in accordance with an embodiment of the disclosure.

Referring to FIG. 3, the ultrasonic probe 100 according to an example includes: the input unit 20 for receiving the user's input command, the ultrasonic probe 100 and a storage unit 30 for storing algorithms and data related to the operation of the incision unit 110, the ultrasonic image acquisition unit 40 for generating an internal image of the object, the adjustment unit 50 for adjusting the incision unit110, the power supply unit 60 for supplying power to each configuration of the ultrasonic probe 100, the display unit 70 for outputting an adjustment result of the incision unit 110, a communication unit 80 for exchanging data with the ultrasonic diagnostic apparatus 10, and a controller 90 for controlling each configuration of the ultrasonic probe 100.

Specifically, the input unit 20 receives the user's input command and transmits an input command to the controller 90. The input unit 20 can receive an initiation command for the ultrasonic probe 100 to irradiate an ultrasonic signal to a target object, an input command for adjusting the depth or angle of the incision unit 110, and a command for setting a region of interest.

The input unit 20 may include a hardware device such as various buttons or switches and a track-ball for receiving an input command, and according to an example, the hardware device may be provided in the lower housing 122 of the ultrasonic probe 100.

The storage unit 30 stores an algorithm related to the operation of the ultrasonic probe 100, image data generated by the ultrasonic image acquisition unit 40, and various data processed by the controller 90.

The storage unit 30 stores data in at least one of a non-volatile memory element such as a cache, ROM (Read Only Memory), PROM (Programmable ROM), EPROM (Erasable Programmable ROM), EEPROM (Electrically Erasable Programmable ROM), and flash memory; a volatile memory element such as RAM (Random Access Memory); or a storage medium such as a hard disk drive (HDD) and CD-ROM, but is not limited thereto.

The ultrasonic image acquisition unit 40 may include the transducer 42 and the image processing unit 44.

As described above, the transducer 42 irradiates an ultrasonic signal and receives the ultrasonic signal reflected from the object. The image processing unit 44 generates an image of the inside of the object based on the electrical signal converted by the transducer 42. The image processing unit 44 transmits the generated image to the controller 90.

There are various ways in which the image processing unit 44 generates images of the inside of the object. Here, the image processing unit 44 transmits the object's internal image to the controller 90 so that the incision unit 110 can be controlled.

On the other hand, the image processing unit 44 is not necessarily provided in the ultrasonic probe 100, and may be provided in the ultrasonic diagnostic apparatus 10. In this case, the controller 90 can transmit the image of the inside of the object via the communication unit 80.

The adjustment unit 50 adjusts the incision unit 110. The adjustment unit 50 includes an angle adjustment unit 52 for adjusting the angle of the incision unit 110 and a depth adjustment unit 54 for adjusting the degree of protrusion of the incision unit 110 from the cover housing 126.

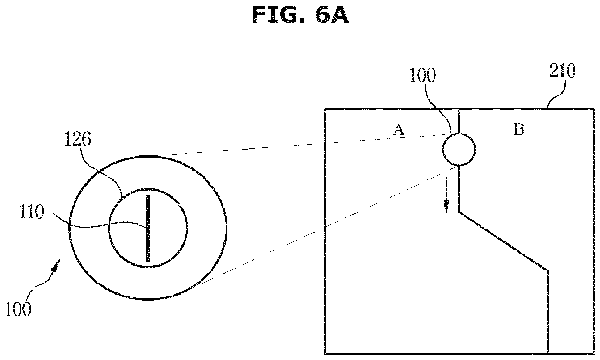

The angle adjustment unit 52 adjusts the angle of the incision unit 110. For example, when the incision unit 110 is a knife, the controller 90 determines the direction in which the ultrasonic probe 100 moves based on the region of interest. If it is determined that the incision direction needs to be adjusted based on the moving direction of the ultrasonic probe, the controller 90 may control the angle adjustment unit 52 to adjust the angle of the knife. Specific explanations related to this will be described later with reference to FIGS. 6A and 6B.

The depth adjustment unit 54 adjusts the depth of the incision unit 110 to insert the incision unit 110 on the surface of the object. For example, the controller 90 may determine that it is possible to avoid a hazardous object or a region of interest that should not be incised in the direction of the movement of the incision unit 110. At this time, the controller 90 controls the depth adjustment unit 54 to adjust the height of the incision unit 110.

The adjustment unit 50 includes a hardware device such as hydraulic, air pressure or a motor. A detailed description related thereto will be described later with reference to FIGS. 5A and 5B.

Meanwhile, the disclosed ultrasonic probe 100 may include various types of the incision units 110.

If the incision unit 110 is formed as a knife, the knife incises the object by the user's work force to operate the ultrasonic probe 100. In this case, the angle adjustment unit 52 or the depth adjustment unit 54 adjusts the degree to which the knife protrudes and the angle of the knife.

As another example, the incision unit 110 may be provided with an electrode made of a material having a low electrical resistance such as iron, and may cut the object through electrical energy or high frequency energy. That is, the incision unit 110 may be configured in the form of an electric scalpel. In this case, the adjustment unit 50 may control the depth or angle at which the object is cut by controlling the current or high frequency output of the current flowing through the electrode.

Specifically, the electric scalpel is a device used in surgery to reduce bleeding during incision. When an electric current flows through the object, it does not give off an electric shock or stimulation to muscles. The electric scalpel is in the form of a scalpel using the principle of sparking or generating heat. The electric scalpel can be classified into an electrosurgical instrument and a high frequency surgical instrument depending on the magnitude of the current flowing through the electrode and the magnitude of the frequency.

If the incision unit 110 is constituted by an electrode for generating a high-frequency current, the incision unit 110 receives electric power from the power supply unit 60, to be described later, and flows a current to the incision unit of the object. In this case, the incision unit 110 discharges a sine high-frequency current of 0.3 MHz to 5 MHz to tissues of the object, and the tissues of the object undergo a boiling rise accompanied by local high pressure due to electron collision. The pressure of the incision site is instantaneously lowered by boiling. As a result, water in the tissue is exploded by joule heat, and the contact area of the electrode is incised.

On the other hand, if the incision unit 110 is constituted by an electrode of the electric scalpel, the incision unit 110 may solidify the object. If the high-frequency current is intermittently flowed through the electrode, the current is cut off before a steam explosion occurs, and the protein of the object is denatured and the region coagulates. That is, the user of the disclosed ultrasonic probe 100 may control the output of the controller 50 through the input unit 20 to solidify the object.

In addition, the incision unit 110 may have a shape for cutting or solidifying a target object through various methods such as an argon beam electric scalpel spraying argon gas from an electrode, CO2 or ND / YAG laser, and it is sufficient if the depth or the angle of the incision site is adjusted on the basis of the image of the incision, and there is no limitation on the manner and form of incision of the incision unit 110.

The power supply unit 60 supplies power to each configuration of the ultrasonic probe 100. Specifically, the power supply unit 60 supplies electric power to the adjustment unit 50 for controlling the incision unit 110, and supplies power to the ultrasonic image acquisition unit 40 to irradiate ultrasonic waves through the transducer 42.

When the ultrasonic probe 100 is connected to the ultrasonic diagnostic apparatus 10 through a wire, the power supply unit 60 serves as a gateway for transmitting power supplied from the ultrasonic diagnostic apparatus 10. However, when the ultrasonic probe 100 is connected to the ultrasonic diagnostic apparatus 10 via radio waves, the power supply unit 60 receives electric power from a portable electric power supply device such as a battery or the like, and transfers the electric power to each configuration.

The display unit 70 outputs a control result of the ultrasonic probe 100.

Specifically, the display unit 70 outputs the adjusted result of the incision unit 110 so that the user can know the adjusted depth or angle of the incision unit 110. The display unit 70 according to an example may be provided in the lower housing 122 of the ultrasonic probe 100.

On the other hand, the display unit 70 may output the image inside the object generated by the ultrasonic image acquisition unit 40, and there is no limitation.

The display unit 70 may be a simple digital display device for displaying the depth to which the incision unit 110 is adjusted and may be a digital light processing (DLP) panel, a plasma display panel, a liquid crystal display (LCD) panel, an electroluminescence (EL) panel, an electrophoretic display (EPD) panel, an electrochromic display (ECD) panel, a light emitting diode (LED) panel or an organic light emitting diode (OLED) panel.

The communication unit 80 transmits ultrasonic signals to the ultrasonic diagnostic apparatus 10 or transmits the generated ultrasonic signals to the ultrasonic probe 100. The communication unit 80 may also receive a control command of the ultrasonic probe 100 from the ultrasonic diagnostic apparatus 10.

On the other hand, the ultrasonic probe 100 may control the incision unit 110 through the communication unit 80, although the controller 90 can adjust the incision unit 110 by itself. For example, if the ultrasonic probe 100 is provided in the slave device such as a surgical robot arm, the incision unit 110 may be controlled by a command transmitted from the master device.

The communication unit 80 may include one or more components that enable communication with the outside of the ultrasonic probe 100, and may include at least one of a short-range communication module, a wired communication module, and a wireless communication module.

The short-range communication module uses a wireless communication network, such as a Bluetooth module, an infrared communication module, an RFID (Radio Frequency Identification) communication module, a WLAN (Wireless Local Access Network) communication module, an NFC communication module, and a Zigbee communication module, and may include various short-range communication modules for transmitting and receiving.

The wired communication module may include various wired communication modules such as a local area network (LAN) module, a wide area network (WAN) module, a value added network (VAN), a high definition multimedia interface (HDMI), a digital visual interface (DVI), recommended standard 232 (RS-232), power line communication, or a plain old telephone service (POTS).

In addition to a Wi-Fi module and a wireless broadband module, the wireless communication module may be GSM (Global System for Mobile Communication), CDMA (Code Division Multiple Access), WCDMA (Wideband Code Division Multiple Access), Time Division Multiple Access (TDMA), Long Term Evolution (LTE), and the like.

The wireless communication module may include a wireless communication interface including an antenna and a transmitter or a receiver for transmitting and receiving signals to be transmitted by the ultrasonic probe 100. The wireless communication module may further include a signal conversion module for modulating and demodulating a digital control signal output from the controller 90 through the wireless communication interface into an analog type wireless signal under the control of the controller 90.

The controller 90 refers to a processor that controls the overall operation of the ultrasonic probe 100. That is, the controller 90 includes a memory (not shown) for storing data for a program reproducing an algorithm or an algorithm for controlling the operation of the components in the ultrasonic probe 100, and a memory (not shown) for performing the above-described operations. At this time, the memory and the processor may be implemented as separate chips. Alternatively, the memory and the processor may be implemented on a single chip.

The controller 90 controls the operation of the adjustment unit 50 according to the region of interest, specifically the depth of the layer of interest, which the incision unit 110 intends to incise, based on the inside of the object generated by the ultrasonic image processing unit 40.

The controller 90 may control the controller 50 to adjust the angle of the incision unit 110 according to the incision area based on the direction in which the ultrasonic probe 100 moves. The detailed description related to the operation of the controller 90 will be described later in detail with reference to the drawings.

The controller 90 may generate an image of the inside of the object through the ultrasonic signal received by the transducer 42 or may convert the ultrasonic signal received by the transducer 42 into an electric signal to be transmitted to the ultrasonic diagnostic apparatus 10.

The configuration shown in FIG. 3 is merely an example of the ultrasonic probe 100 according to an exemplary embodiment, and may include various configurations.

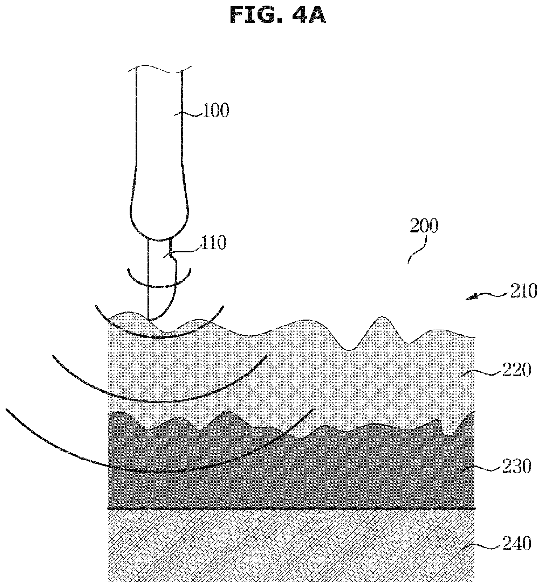

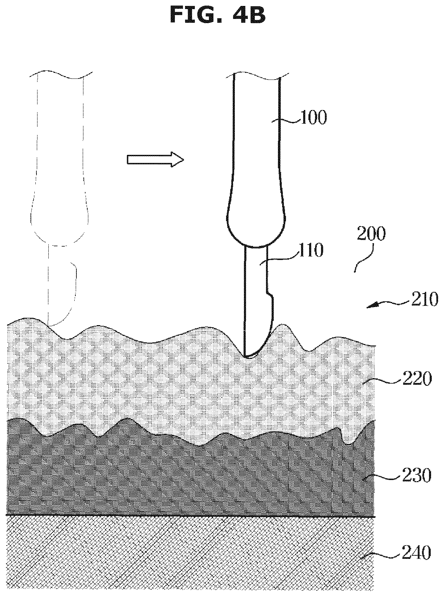

FIGS. 4A and 4B are views illustrating an operation of an ultrasonic probe in accordance with one embodiment. To avoid redundant explanations, the following will be described together.

Referring to FIG. 4A, the ultrasonic probe 100 irradiates ultrasonic wave to an object 200 and determines the interior of the object 200 through the reflected ultrasonic signal.

The interior of the object 200 according to an exemplary embodiment may include a skin layer 210, a dermal layer 220, a subcutaneous fat layer 230, and a muscle layer 240. The user can set the depth of the region of interest to be incised to the depth of the skin layer 210. In this case, the ultrasonic probe 100 may control the incision unit 110 to protrude up to the depth of the skin layer 210.

Referring to FIG. 4B, the ultrasonic probe 100 can move in the direction of an arrow. As the ultrasonic probe 100 moves, the object 200 can be incised by the incision unit 110.

The ultrasonic probe 100 continuously examines the ultrasonic signal while moving, and continuously determines the interior of the object 200. At this time, the ultrasonic probe 100, specifically, the controller 90 can recognize that the depth of the skin layer 210 corresponding to the region of interest changes based on the generated image. In this case, the controller 90 can adjust the depth of the incision unit 110 as shown in FIG. 4B so that the incision unit 110 is incised only to a predetermined region of interest, that is, the skin layer 210.

As shown in FIG. 4B, the ultrasonic probe 100 can control the incision unit 110 to protrude further from the cover housing 126. The ultrasonic probe 100 prevents the dermal layer 220 under the skin layer 210 from being incised and can prepare for a dangerous situation that may occur when the dermal layer 220 is incised.

Meanwhile, FIGS. 4A and 4B are merely examples of the operation of the disclosed ultrasonic probe 100. As another example, if the user is set to insert the incision unit when there is another dangerous object such as a blood vessel in the incision object, the ultrasonic probe 100 may stop the incision by inserting the incision unit 110 completely into the inside of the incision part.

FIGS. 5A and 5B are views illustrating a method of adjusting an incision unit in accordance with one embodiment.

Specifically, in FIGS. 5A and 5B, the incision unit 110 and the adjustment unit 50 may be provided in one aspect thereof. That is, the ultrasonic probe 100 has the incision unit 110 inserted into the cover housing 126 in FIGS. 4A and 4B, and the following ultrasonic probe 100 has a shape in which the controller 50 and the incision unit 110 are attached to one side of the cover housing 126.

Referring to FIG. 5A, the adjustment unit 50 according to an exemplary embodiment of the present disclosure, the adjustment unit 50, specifically the depth adjustment unit 54, may adjust the depth of the incision unit 110 through a piston 56 connected to one end of the incision unit 110.

The depth adjustment unit 54 can adjust a piston 55 up or down through hydraulic or air pressure. That is, the piston 55 is moved up and down by the change of the hydraulic pressure or the air pressure, and the depth of the incision unit 110 connected to the piston 55 is adjusted.

Referring to FIG. 5B, the depth adjustment unit 54 according to another example may move the incision unit 110 using a motor (not shown). Specifically, the motor rotates a pinion 57 engaged with a rack 58. The rotational movement of the pinion 57 is converted into a linear movement by the rack 58. One end of the rack 58 is provided with the incision unit 110 and the depth of the incision unit 110 can be adjusted by the linear movement of the rack 58.

On the other hand, FIGS. 5A and 5B can be applied to the case where the adjustment unit 50 and the incision unit 110 are provided inside the ultrasonic probe 100 according to an example. In addition, the example in which the adjustment unit 50 adjusts the incision unit 110 is not necessarily limited to those shown in FIGS. 5A and 5B, and various modifications may be made.

FIGS. 5A and 5B are views illustrating another operation of an ultrasonic probe in accordance with one embodiment. To avoid redundant explanations, the following will be described together.

FIGS. 6A and 6B are views in which the incision unit 110 is viewed from below the ultrasonic probe 100 provided inside the cover housing 126. In addition, the region of interest of the object 200 to be dissected may be divided into regions A and B, respectively.

First, as shown in FIG. 6A, the ultrasonic probe 100 moves so that the region A and the region B are orthogonal to each other, and the incision unit 110 can incise the object in a straight line shape according to the movement of the ultrasonic probe 100.

The ultrasonic probe 100 irradiates an ultrasonic wave to a target object while moving, and it can be determined that it is necessary to adjust the angle of the incision unit 110 as shown in FIG. 6B based on the object and the region of interest corresponding to the reflected ultrasonic signal. In this case, the controller 90 controls the angle adjustment unit 52 to adjust the angle of the incision unit 110 as shown in FIG. 6B. As a result, the incision unit 110 is rotated and the object can be incised depending on the region of interest.

Meanwhile, FIG. 6B shows an example in which the angle of the incision unit 110 is adjusted, and the angle of the incision unit 110 can be variously adjusted. Also, the ultrasonic probe 100 may display the result of the adjustment of the incision unit 110 on the display unit 70, and the user may readjust the adjusted angle through the input unit 20.

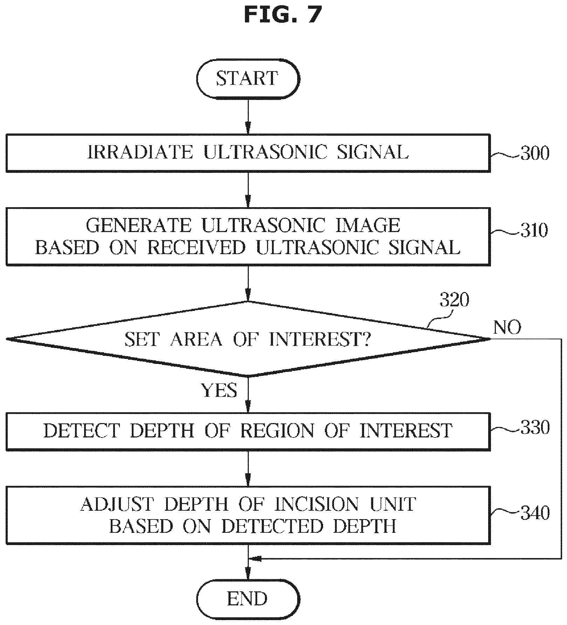

FIG. 7 is a flowchart of a method of adjusting a depth of an incision unit by an ultrasonic probe in accordance with one embodiment.

Referring to FIG. 7, the ultrasonic probe 100 irradiates an ultrasonic signal to a target object (300).

Specifically, in the ultrasonic probe 100, the transducer 42 irradiates an ultrasonic signal.

The ultrasonic probe 100 receives the ultrasonic signal reflected from the target object, and generates an image of the interior of the object based on the reflected signal (310).

The user may set an area of interest (320) for setting the depth of incision of the incision unit 110 based on the image of the inside of the object generated by the ultrasonic probe 100.

The region of interest may be a predetermined depth or may be set by the input unit 20.

Once the region of interest is set, the ultrasonic probe 100 detects the depth of the region of interest based on the ultrasonic image within the object (330).

The depth of the region of interest may vary, such as in FIG. 5A. In this case, the ultrasonic probe 100 adjusts the height of the incision unit 110 according to the depth of the region of interest continuously received by the ultrasonic probe 100 while moving (340).

The disclosed ultrasonic probe 100 can cut the object only up to the depth of the region of interest.

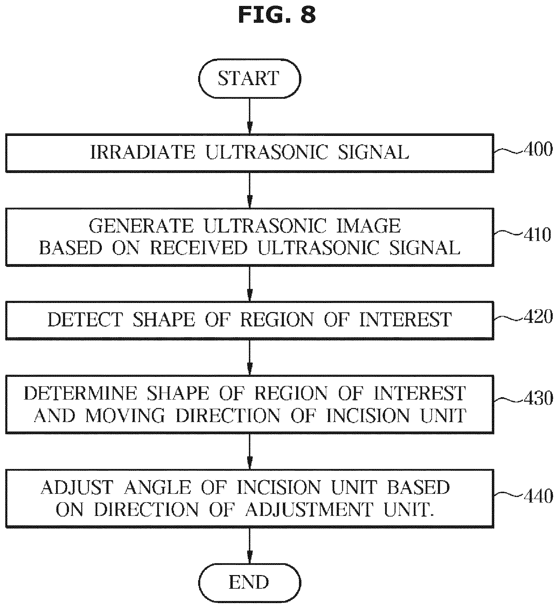

FIG. 8 is a flowchart of a method of adjusting an angle of an incision unit by an ultrasonic probe in accordance with one embodiment.

Referring to FIG. 8, the ultrasonic probe 100 irradiates an ultrasonic signal to a target object (400).

Specifically, in the ultrasonic probe 100, the transducer 42 irradiates an ultrasonic signal.

The ultrasonic probe 100 receives the ultrasonic signal reflected from the target object, and generates an image of the target based on the reflected signal (410).

The ultrasonic probe 100 detects the shape of the object, that is, the shape of the region of interest based on the generated image (420).

The shape of the region of interest may be varied as shown in FIG. 6A. In addition, the shape of the region of interest can be set in advance. The user may change the shape of the region of interest set based on the image generated while the ultrasonic probe 100 moves.

The ultrasonic probe 100 determines the shape and moving direction of the detected region of interest (430).

Specifically, the controller 90 of the ultrasonic probe 100 determines the shape and moving direction of the region of interest.

The controller 90 controls the adjustment unit 50 based on the determined shape and moving direction of the region of interest.

Specifically, when the shape of the region of interest to be cut varies according to the moving direction, the adjustment unit 50 adjusts the angle of the incision unit 110 so that the object can be incised in the shape of the region of interest.

If the direction of movement of the ultrasonic probe 100 is out of the predetermined range by the user, the controller 90 inserts the incision unit 110 into the ultrasonic probe 100 or changes the angle of the incision unit 110 again.

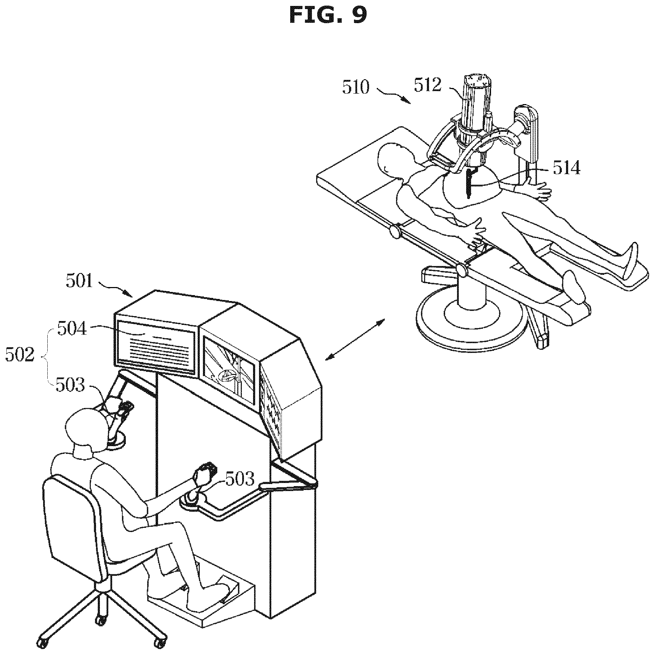

FIG. 9 is a view illustrating an example in which the disclosed ultrasonic probe according to another embodiment is applied to a surgical robot system.

The surgical robot according to the embodiment inserts a robot arm into the body of a patient to perform surgery.

Referring to FIG. 9, the surgical robot system includes a slave robot 510 for performing surgery on the patient lying on the operating table, and a master console 501 for remotely operating the slave robot 510.

The master console 501 and the slave robot 510 are not necessarily separated from each other by a separate device that is physically independent and may be integrated into one unit. In this case, a master interface 502 may correspond to, for example, an interface part.

The master interface 502 of the master console 501 includes a monitor unit 504 and a master controller and the slave robot 510 includes a slave arm 512 and a robot arm 514. The robot arm 514 is an operation tool such as an endoscope such as a laparoscope or the like, a surgical operation part for operating the affected part directly, or the like. Hereinafter, the surgical robot arm 514 includes the ultrasonic probe 100 including the incision unit 110 and will be described with reference to a situation in which the robot arm 514 is inserted into an object to cut the object.

The master interface 502 is provided with the master controller so that an operator can be grasped and manipulated by both hands, respectively. The master controller may be implemented with two handles 503 as illustrated in FIG. 9, and an operation signal according to the operation of the operator's handles 503 is transmitted to the slave robot 510 to control the slave arm 512. The slave arm 512 and / or the robot arm 514 can be moved, rotated, cut, or the like by operating the handles 503 of the operator.

For example, a handle 513 may comprise a main handle and a sub handle. The slave arm 512, the robot arm 514, and the like may be operated with only one handle, or a plurality of surgical instruments may be simultaneously operated in real time by adding the sub handle. The main handle and the sub handle may have various mechanical configurations according to their operation modes, for example, various input means may be used to operate the slave arm 512 of the slave robot 510 and / or other surgical equipment, such as a joystick form, a keypad, a trackball, a touch screen, and the like.

The master controller is not limited to the shape of the handles 503, and can be applied without any limitation as long as it controls the operation of the slave arm 512 through a network. The robot arm 514 is mounted on a distal end portion of the surgical slave arm 512 provided with an actuator, and the driving force is received from the actuator of the slave robot 510 and the robot arm 514 is operated to incise the object.

The monitor unit 504 of the master interface 502 displays an image of the inside of the object transmitted by the ultrasonic probe 100 provided in the robot arm 514 as an image. The user sets the region of interest through which the object is to be incised through the displayed image and the robot arm 514 can adjust the depth or angle of the incision unit 110 included in the ultrasonic probe 100 based on the region of interest.

The slave robot 510 and the master console 501 are coupled to each other through a wired communication network or a wireless communication network, and an operation signal, an endoscopic image input through the robot arm 514, and the like can be transmitted to another party. If two operation signals by the two handles 503 provided in the master interface 502 and / or an operation signal for adjusting the position of the robot arm 514 need to be transmitted at the same time and /or at a similar time, each of the operation signals can be transmitted to the slave robot 510 independently of each other. Here, the fact that each of the operation signals is transmitted 'independently' means that the operation signals do not interfere with each other, and that any one of the operation signals does not affect the other. Therefore, when the master console 501 generates operation signals for controlling the surgical robot arm 514 and the surgical robot, the respective operation signals are transmitted to the slave robot 510 independently of each other, and the actuator coupled to the apparatus can be driven.

In order to allow a plurality of the operation signals to be independently transmitted, header information for each of the operation signals is added and transmitted in the generation of each of the operation signals, or each of the operation signals is transmitted in accordance with the generation order, or a variety of schemes may be used, such that priorities are given in advance regarding the transmission order of each of the operation signals and are transmitted in accordance with the priorities. In this case, a transmission path through which each of the operation signals is transmitted may be provided independently so that interference between each of the operation signals may be fundamentally prevented.

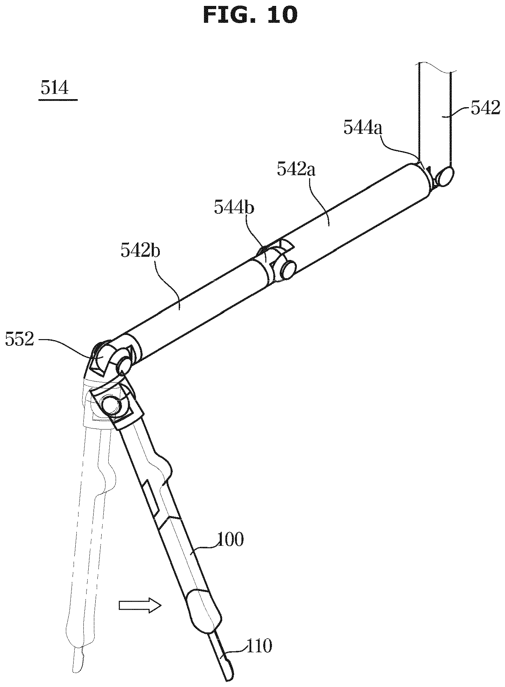

FIG. 10 is a view illustrating an operation of a robot arm.

The disclosed ultrasonic probe 100 is provided in the robot arm 514 of the slave robot 510 and can operate as shown in FIG. 10 according to a control signal of the master console 501.

The robot arm 514 may be provided at the end of the slave robot 510 and may include a plurality of arm portions. The arm unit 514 can be unfolded in different directions and can be focused in one direction. To this end, the arm unit 514 engages between shoulder joints 544a, 544b and the shoulder joints 544a, 544b, and may include shoulder links 542, 542a and 542b on which the ultrasonic probe 100 is supported.

The shoulder links 542a and 542b allow the robot arm 514 to incise the object while rotating in various directions.

An elbow joint 552 provided at an end of the robot arm 514 connects the ultrasonic probe 100 with the shoulder link 542b, and the incision unit 110 moves the ultrasonic probe 100 according to a direction in which the object is incised.

As shown in FIG. 10, the elbow joint 552 can be adjusted to incise the object while moving the ultrasonic probe 100.

Meanwhile, the ultrasonic probe 100 according to the illustrated example irradiates an ultrasonic signal to a target while moving according to the rotation of the elbow joint 552, and receives the reflected ultrasonic signal. The ultrasonic probe 100 moves according to the control of the robot arm 514 and adjusts the height or angle of the incision unit 110 based on the inside of the object generated by the received ultrasonic signal.

As shown in FIG. 10, the ultrasonic probe 100 moving in the direction of the arrow adjusts the height of the incision unit 110 before the movement to protrude longer. The surgical robot system disclosed in the above description can reduce the inconvenience of the user repeatedly adjusting the incision unit 110 when the robot arm 514 moves, and helps the incision member to incise a desired area.

[Industrial Applicability]

[Sequence List Text]

## Claims

1. An ultrasonic probe, comprising:
an incision unit configured to incise a target object;
an adjustment unit configured to adjust the incision unit;
a transducer configured to irradiate an ultrasonic signal to the target object and receive the reflected ultrasonic signal;
a controller configured to determine the inside of the target object based on the delivered signal by the transducer and control the adjustment unit corresponding to the inside of the target object.

2. The ultrasonic probe of claim 1, wherein the controller sets a region of interest in which the inside of the target object is incised.

3. The ultrasonic probe of claim 1, wherein the controller controls the adjustment unit based on a depth of the inside of the target object.

4. The ultrasonic probe of claim 1, wherein the controller controls the adjustment unit to adjust an angle of the incision unit based on an incision direction of the inside of the target object.

5. The ultrasonic probe of claim 1, wherein
the adjustment unit further comprising a piston configured to be connected to the incision unit; and wherein
the controller controls hydraulic or air pressure inside the adjustment unit to move the piston.

6. The ultrasonic probe of claim 1,
the adjustment unit comprising:
a rack configured to be connected to the incision unit;
a pinion configured to be located on the rack, and rotate; and
a motor configured to rotate the pinion; and
wherein
the controller controls the adjustment unit to adjust the depth of the incision unit by driving the motor.

7. The ultrasonic probe of claim 1, wherein
the incision unit is disposed at one end of a housing of the ultrasonic probe.

8. The ultrasonic probe of claim 1, wherein
the incision unit is located at the center of a cover housing of the ultrasonic probe.

9. The ultrasonic probe of claim 1, further comprising:
a display unit configured to display an adjusting result of the incision unit.

10. The ultrasonic probe of claim 1, further comprising:
an input unit to receive a user's input command, and
wherein the controller controls the adjustment unit based on the delivered signal from the input unit.

11. A method for controlling an ultrasonic probe comprising an incision unit, the method comprising:
irradiating an ultrasonic signal to a target object and receiving the reflected ultrasonic signal;
determining the inside of the target object based on the received ultrasonic signal; and
adjusting an adjustment unit corresponding to the inside of the target object.

12. The method of claim 11,
wherein the determining comprises,
setting a region of interest in which the inside of the target object is incised.

13. The method of claim 11,
wherein the adjusting comprises,
adjusting a depth of the incision unit.

14. The method of claim 11,
wherein the adjusting comprises,
adjusting an angle of the adjustment unit based on an incision direction of the target object.

15. The method of claim 13,
wherein the adjusting comprises,
adjusting at least one of hydraulic pressure, air pressure and a motor for moving a piston connected to the incision unit.
